# EUROPÄISCHE PATENTSCHRIFT

(11) **EP 2 919 732 B1**
(45) Veröffentlichungstag und Bekanntmachung des Hinweises auf die Patenterteilung: **08.12.2021**
(21) Anmeldenummer: 13783884.3
(22) Anmeldetag: 21.10.2013
(51) Int. Cl.: A61F 13/15, B65B 35/00, B65B 9/067

(54) **ANLAGE ZUR HERSTELLUNG VON VERPACKTEN PRODUKTSTAPELN, INSBESONDERE VON HYGIENEPRODUKTEN**
INSTALLATION FOR THE PRODUCTION OF PACKED PRODUCT STACKS, ESPECIALLY OF HYGIENIC PRODUCTS
INSTALLATION POUR LA FABRICATION DE PILES DE PRODUITS EMBALLÉES, NOTAMMENT DE PRODUITS HYGIÉNIQUES

(30) Priorität: 14.11.2012 DE 102012024593
(43) Veröffentlichungstag der Anmeldung: 23.09.2015
(73) Patentinhaber: Winkler + Dünnebier GmbH, 56564 Neuwied (DE)
(72) Erfinder: RINKE, Andreas, 23843 Bad Oldesloe (DE); MAJEWSKI, Rolf, 53579 Erpel (DE); POPP, Konrad, 86179 Augsburg (DE); JUNGBLUTH, Frank, 53506 Arbrück (DE); TEWS, Thorsten, 56237 Kruft (DE); BRETZ, Michael, 56567 Neuwied (DE)
(74) Vertreter: Walkenhorst, Andreas
(86) Internationale Anmeldenummer: PCT/EP2013/071957
(87) Internationale Veröffentlichungsnummer: WO 2014/075872

(56) Entgegenhaltungen:
- DE-A1- 10 344 335
- US-B1- 6 189 827

## Beschreibung

Die Erfindung betrifft eine Anlage zur Herstellung von verpackten Produktstapeln von gefalzten oder ungefalzten Produktzuschnitten aus Papier, Zellstoff oder dergleichen, insbesondere von Hygieneprodukten, mit mindestens zwei produktstromseitig parallelgeschalteten Staplereinheiten zum Bilden der Produktstapel aus den Produktzuschnitten, denen produktstromseitig über ein Fördersystem eine gemeinsame Verpackereinheit nachgeschaltet ist.

Eine derartige Anlage ist beispielsweise aus der DE 103 44 335 A1 bekannt.

In einer Vielzahl von Anwendungen, beispielsweise in der papierverarbeitenden Industrie oder auch bei der Herstellung von Hygieneprodukten, werden Produktzuschnitte aus Papier, Zellstoff, Vliesstoff oder dergleichen hergestellt. Die Produktzuschnitte können dabei in ungefalztem Zustand oder auch in gefalztem Zustand wie beispielsweise bei der Herstellung von Briefumschlägen aus Papier oder bei der Herstellung von Taschentüchern oder dergleichen, aus Zellstoff vorliegen. Derartige Produkte, insbesondere Hygieneprodukte, werden üblicherweise in besonders großer Stückzahl hergestellt und nach der Faltung oder Falzung in geeignet gewählten Packungsgrößen, beispielsweise in 5er- oder 10er-Packs, verpackt. Dazu werden in geeigneten Verpackungssystemen zunächst geeignet dimensionierte Produktstapel, also abhängig vom vorgesehenen Einsatzzweck beispielsweise 5er ― oder 10er Produktstapel, aus den Zuschnitten gebildet und anschließend dem eigentlichen Verpacker zugeführt, wo sie beispielsweise in eine Beutelschlauchfolie oder dergleichen eingeschlagen werden.

Gerade beim Verpacken von Hygieneprodukten, die in besonders großer Stückzahl hergestellt werden, wie beispielsweise Papiertaschentücher, Slipeinlagen, Damenbinden oder dergleichen, ist eine hohe Bearbeitungsgeschwindigkeit und Durchsatzrate beim Verpacken der Produkte wünschenswert. Aus diesem Grund werden die Verpackungssysteme üblicherweise in der Art von Durchlaufsystemen ausgelegt. Bei diesen werden die zu verpackenden Produkte nach der Stapelbildung in aufeinanderfolgenden Produktstapeln entlang einer Transportrichtung weiterbefördert und während des Durchlaufs durch verschiedene, entlang der Transportrichtung angeordnete Systemkomponenten mit dem Verpackungsbeutel umgeben. Insbesondere die genannten Folienschlauchsysteme sind für hohe Bearbeitungsgeschwindigkeiten und Durchsatzraten grundsätzlich gut geeignet, da beispielsweise beim Einschlagen der Produkte oder Produktstapel in die Schlauchfolie zur Bildung des Folienschlauchs ein Verschweißen der seitlichen Längsnaht von der Seite her, also im Durchlauf und ohne Systemstillstand, möglich ist. Um einen zuverlässigen Betrieb eines derartigen Systems gerade auch bei hohen Stückzahlen gewährleisten zu können, ist aber üblicherweise eine so genannte register- oder taktgenaue Zuführung der Produktstapel in den Verpacker erforderlich, bei der die Produktstapel in gleichmäßigen, geeignet an den folgenden Verpackungsprozess angepassten Abständen zueinander angeliefert werden.

Wenn diese Voraussetzungen geeignet eingehalten werden, sind vergleichsweise hohe Durchsatzraten im Verpacker erreichbar. Die den Durchsatz der Gesamtanlage begrenzende Größe ist dann die Produktionsrate der dem Verpacker produktstromseitig vorgeschalteten Staplereinheit, in der aus den einzelnen Produktzuschnitten die später zu verpackenden Produktstapel zu bilden. Für die Gesamtanlage ist es daher im Hinblick auf die angestrebten hohen Durchsatzraten wünschenswert, dem Verpacker eine Mehrzahl von produktstromseitig parallel geschalteten Staplereinheiten vorzuschalten. Um dabei aber dennoch die für den ordnungsgemäßen Prozessablauf im Verpacker erforderliche registergenaue Zuführung der Produktstapel einzuhalten, ist eine geeignet abgestimmte und synchronisierte Überführung der Produktstapel von den Staplereinheiten zum Verpacker erforderlich.

Um dem Rechnung zu tragen, werden die Produktstapel üblicherweise von mit konstanter Transportgeschwindigkeit laufenden Ketten- oder Bandtransportsystemen aus den Stapelbereichen der Staplereinheiten aufgenommen und abtransportiert. Durch Schrägstellung dieser Bänder oder Ketten erfolgt die Vereinigung der Produkt-Teilströme in einer Bahn. Um dabei eine für den nachfolgenden Verpacker ausreichend genaue registerhaltige Teilung oder einen registerhaltigen Abstand der Produktstapel einzuhalten, erfolgt eine Umlenkung der Produktstapel um 90°, wobei sie aus der Umlenkstelle in der entsprechend registergenauen Teilung abgeführt werden. Dazu wird der jeweilige Produktstapel vergleichsweise hart gegen einen Anschlag gefahren, um dann mit einer Fördereinrichtung in einer Richtung um 90° zur ursprünglichen Richtung abtransportiert zu werden. Die Mitnehmer der Fördereinrichtung haben dabei die für den Verpacker notwendige Teilung.

Ein derartiges System ist jedoch apparativ vergleichsweise aufwendig und setzt die Produktstapel zudem einer vergleichsweise hohen mechanischen Belastung aus. Insbesondere bedingen die mit konstanter Transportgeschwindigkeit laufenden Bänder oder Ketten für die zu fördernden Produktstapel hohe stoß- und ruckartige Bewegungen. Durch die schräg laufenden Bänder werden die Produktstapel zudem trapezartig verzerrt. Dies kann insbesondere bei der Verarbeitung von Hygieneprodukten auf Zellstoffbasis eine unerwünscht hohe mechanische Belastung der Produktstapel bedingen, und die genannten Umlenkungen erfordern in der Regel apparativ aufwendige Saugsysteme zum vorübergehenden Festhalten der Produktstapel.

Der Erfindung liegt daher die Aufgabe zugrunde, eine Anlage der oben genannten Art anzugeben, mit der unter Vermeidung der genannten Nachteile eine besonders hohe Durchsatz- und Produktionsrate erreichbar ist.

Diese Aufgabe wird erfindungsgemäß gelöst, indem die Anlage mindestens zwei produktstromseitig parallelgeschalteten Staplereinheiten zum Bilden der Produktstapel aus den Produktzuschnitten umfasst, denen produktstromseitig über ein Fördersystem eine gemeinsame Verpackereinheit nachgeschaltet ist, wobei das Fördersystem einen ersten, einer der Staplereinheiten zugeordneten einteiligen Förderer und einen zweiten, einer zweiten Staplereinheit zugeordneten mehrteiligen Förderer umfasst.

Vorteilhafte Ausgestaltungen der Erfindung sind Gegenstand der Unteransprüche.

Die Erfindung geht von der Überlegung aus, dass für die angestrebten hohen Produktions- und Durchsatzraten dem Verpacker eine Mehrzahl von Staplereinheiten vorgeschaltet sein sollte. Das Fördersystem, mit dem die gebildeten Produktstapel von den Staplereinheiten zum Verpacker transportiert werden, sollte dazu gezielt für die gewünschte Vereinigung der Produkt-Teilströme mit einbahniger oder einkanaliger Zufuhr der Produktstapel mit registergenauer Teilung zum Verpacker ausgelegt sein.

Für eine der Staplereinheiten ist dabei eine einteilige Ausgestaltung des zugeordneten Förderers in dem Sinne vorgesehen, dass dieser in Transportrichtung des Produktstroms gesehen lediglich eine Fördereinheit aufweist, die den vollständigen Transport der Produktstapel von der Stapeleinheit bis hin zum Verpacker bewerkstelligt. Die Fördereinheit ist dabei vorzugsweise mit einem ansteuerbaren oder programmierbaren Servoantrieb versehen, so dass die Transportgeschwindigkeit an die Erfordernisse des aktuellen Prozesszustandes anpassbar ist. Für die Ansteuerung oder Programmierung der Fördereinheit ist dabei vorzugsweise - angepasst an die Prozesstaktung - ein Bewegungsprofil vorgesehen, das eine vergleichsweise schonende Behandlung der Produktstapel, insbesondere im Hinblick auf die mechanischen Belastungen, gewährleistet. Vorteilhafterweise ist dazu vorgesehen, dass die Fördereinheit den jeweiligen Produktstapel, beispielsweise über einen Schieber, von der der Staplereinheit nachfolgenden Ablegeposition mit einer zunächst vergleichsweise niedrigen Geschwindigkeit aufnimmt und ihn anschließend beschleunigt. Über eine entsprechende Einstellung der Transportgeschwindigkeit erfolgt sodann eine Anpassung an die relative Position im Produktionsablauf, um damit die erforderliche Registerhaltigkeit zu gewährleisten. Anschließend wird der Produktstapel mit einer an die Fördergeschwindigkeit des Folgeprozesses, also beispielsweise des Verpackers, angepassten Geschwindigkeit an diesen abgegeben.

Das Bewegungsprofil der Fördereinheit ist dabei derart abgestimmt, dass in jeder Phase, also insbesondere auch in der Anschubphase, nur vergleichsweise geringe Stöße auf den Produktstapel erfolgen. Auch evtl. notwendige Verzögerungsphasen beim Staffelvorgang, z. B. im Zusammenhang mit der Übergabe an nachfolgende Systemkomponenten und/oder im Zusammenhang mit der registergenauen Positionierung innerhalb des Produktstroms erfolgen dabei in Längsrichtung des Produktstapels und werden weitgehend schonend durchgeführt. Dazu können in besonders vorteilhafter Ausgestaltung auch zusätzliche Bremssysteme wie beispielsweise Reibungsbremsen oder auch vakuumtechnische Lösungen, beispielsweise ein Saugvakuum zur vorübergehenden und/oder unterstützenden Fixierung oder Führung der Produkte, vorgesehen sein.

Für eine weitere Staplereinheit ist dem gegenüber eine zwei- oder mehrteilige Ausgestaltung des zugeordneten Förderers in dem Sinne vorgesehen, dass dieser in Transportrichtung des Produktstroms gesehen eine Mehrzahl hintereinander angeordneter Fördereinheiten in der Art einer Reihenschaltung aufweist, auf die der vollständige Transport der Produktstapel von der Stapeleinheit bis hin zum Verpacker aufgeteilt ist. Auch diese Fördereinheiten sind dabei vorzugsweise jeweils mit einem Servoantrieb versehen, der vorzugsweise für jede Fördereinheit unabhängig ansteuerbar oder programmierbar ist. Die in Transportrichtung des Produktstroms gesehen erste dieser Fördereinheiten ist dabei hinsichtlich ihres Bewegungsprofils ähnlich wie die vorgenannte Fördereinheit vornehmlich für eine vergleichsweise schonende Annahme und Beschleunigung des jeweiligen Produktstapels bei der Übernahme von der Ablageposition der Staplereinheit ausgelegt. Die nachfolgende Fördereinheit, an die der jeweilige Produktstapel von der vorangehenden Fördereinheit übergeben wird, erfüllt hingegen prozesstechnisch die Funktion eines Puffer- und/oder Synchronisationselements, wobei die Fördergeschwindigkeit derart eingestellt wird, dass gerade auch in Abstimmung mit der Fördereinheit der ersten Staplereinheit eine register- und taktgenaue Vereinigung der Produktströme miteinander möglich ist.

Durch die mehrteilige, vorzugsweise zweiteilige, Ausführung des der zweiten Staplereinheit zugeordneten Förderers ist somit erreicht, dass sowohl eingangsseitig eine taktgenaue Abnahme der von der Staplereinheit angelieferten Produktstapels als auch unabhängig davon ausgangsseitig eine synchronisierte und an den Produktfluss des der ersten Staplereinheit zugeordneten Förderers angepasste und damit registergenaue Abgabe des Produktstapels an die nachfolgenden Systeme möglich ist. Durch die unabhängig voneinander mögliche Einstellung der Transportgeschwindigkeiten kann der zweite Förderer somit sowohl eingangs- als auch ausgangsseitig mit dem Prozessumfeld synchronisiert werden.

Auch für die Ansteuerung der Fördereinheiten des zweiten Förderers ist vorzugsweise - angepasst an die Prozesstaktung - ein Bewegungsprofil vorgesehen, das eine vergleichsweise schonende Behandlung der Produktstapel, insbesondere im Hinblick auf die mechanischen Belastungen, gewährleistet. Vorteilhafterweise ist dazu vorgesehen, dass die in Förderrichtung gesehen erste Fördereinheit des zweiten Förderers ebenfalls den jeweiligen Produktstapel, beispielsweise über einen Schieber, von der der Staplereinheit nachfolgenden Ablegeposition mit einer zunächst vergleichsweise niedrigen Geschwindigkeit aufnimmt und ihn anschließend beschleunigt. Von der in Förderrichtung gesehen zweiten oder letzten Fördereinheit wird der Produktstapel hingegen vorzugsweise mit einer an die Fördergeschwindigkeit des Folgeprozesses, also beispielsweise des Verpackers, angepassten Geschwindigkeit an diesen abgegeben.

Dabei kann vorteilhafterweise vorgesehen sein, auf Bandförderer als Fördersysteme zurückzugreifen. Alternativ können die jeweiligen Fördereinheiten auch als Riementriebe oder dergleichen, vorzugsweise versehen mit Schiebern, ausgestaltet sein.

Die mit der Erfindung erzielten Vorteile liegen insbesondere darin, dass durch die mehrteilige Ausführung zumindest eines der Förderer und geeignete separate und unabhängige Ansteuerung oder Programmierung der jeweiligen Fördereinheiten mit vergleichsweise gering gehaltenem apparativen Aufwand eine zuverlässige Vereinigung der von einer Mehrzahl von Staplereinheiten gelieferten Produktstapel zu einem gemeinsamen Produktstrom mit registergenauem Abstand der Produktstapel zueinander ermöglicht ist. Durch eine geeignete Antriebstechnik, vorzugsweise über programmierbare oder ansteuerbare Servomotoren, kann zudem das Gesamtbewegungsprofil aller Fördereinheiten oder Transportbänder auf die formatspezifischen Erfordernisse oder Details der Produkte und/oder der Stapelmenge abgestimmt werden.

Ein Ausführungsbeispiel der Erfindung wird anhand einer Zeichnung näher erläutert. Darin zeigen:
- FIG. 1: Eine Anlage zur Herstellung von verpackten Produktstapeln in Draufsicht, und
- FIG. 2: schematisch vereinfacht in vergrößerter Ansicht ein Fördersystem der Anlage gemäß FIG. 1.

Gleiche Teile sind in allen Figuren mit denselben Bezugszeichen versehen.

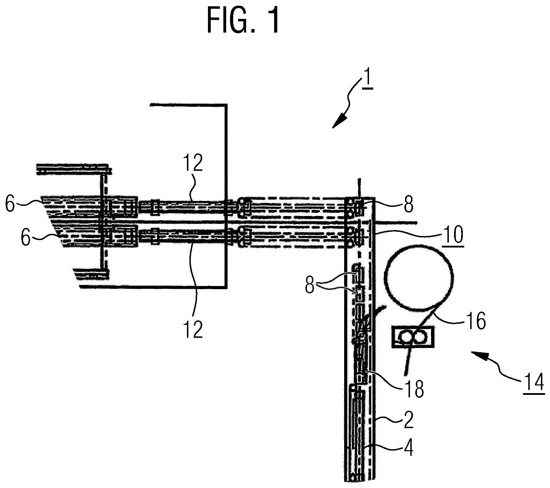

Die Anlage 1 gemäß FIG. 1 ist zur Herstellung von verpackten Produktstapeln von Hygieneprodukten aus Zellstoff, im Ausführungsbeispiel von Papiertaschentüchern, vorgesehen. Im Ausführungsbeispiel sollen dabei als Hygieneprodukte Papiertaschentücher chargenweise, also als Stapel einzelner Taschentücher, mit einer Chargengröße von jeweils zehn Stück in jeweils einen Verpackungsbeutel eingebracht werden. Alternativ könnten selbstverständlich aber auch andere Chargengrößen, beispielsweise die Verpackung von drei oder fünf Papiertaschentüchern in einen Verpackungsbeutel, oder auch andere Hygieneprodukte wie beispielsweise Slipeinlagen, Damenbinden oder dergleichen, vorgesehen sein.

Die Anlage 1 weist dazu eine als Folienschlauch-Verpacker ausgelegte Verpackereinheit 2 auf, in der die zu verpackenden Papiertaschentücher chargen- oder stapelweise zunächst in einen Folienschlauch 4 eingeschlagen werden. Um dabei hohe Produktions- und Durchsatzraten zu erreichen, umfasst die Anlage 1 mehrere, im Ausführungsbeispiel zwei, produktstromseitig parallelgeschaltete und lediglich schematisch angedeutete Staplereinheiten 6 zum Bilden von Produktstapeln 8 aus jeweils zehn übereinander liegenden Papiertaschentüchern. Den Staplereinheiten 6 ist die Verpackereinheit 2 produktstromseitig über ein Fördersystem 10 als gemeinsame Verpackereinheit nachgeschaltet. Das heißt, in der Art einer parallelen Anordnung zweier Zuführschienen 12 werden die Produktstapel 8 parallel zueinander dem Fördersystem 10 zugeführt. Diese zweibahnige Anordnung ermöglicht insbesondere die Zusammenführung mehrerer Transportstränge für eine gemeinsame Weiterverarbeitung der Produktstapel 8. Damit sind - im Vergleich zur Bereitstellungsrate der Produktstapel 8 bei deren Herstellung - verdoppelte Durchsatzraten beim Verpacken und bei der Weiterverarbeitung der Produktstapel 8 erreichbar.

Die Verpackereinheit 2 umfasst u. a. eine Einschlagstation 14, in der in einem geeigneten Folienspeicher, beispielsweise in Form von Rollen oder dergleichen, die zur Bildung der Verpackungsbeutel vorgesehene Schlauchfolie 16 vorgehalten ist. In der Einschlagstation 14 ist ein geeignet geformter Formschuh 18 vorgesehen, über den die Schlauchfolie 16 um die von dem Fördersystem 10 zugeführten Produktstapel 8 gelegt wird, wobei die Papiertaschentücher in die Folie eingeschlagen werden. Der Formschuh 18 ist dabei derart ausgestaltet, dass die zugeführte Schlauchfolie 16 die durchlaufenden Produktstapel 8 vollständig umgibt, wobei an der Längskante des dabei entstehenden Folienschlauchs 4 eine Überlappung der entsprechenden Kantenbereiche der Folie eingestellt wird. Den genannten Komponenten sind produktstromseitig noch weitere nicht näher dargestellte Anlagenteile nachgeschaltet.

Die Einschlagstation 14 ist mit geeigneten Mitteln zur örtlichen Synchronisierung der Produktstapel 8 mit der Schlauchfolie 16 versehen. Dabei ist insbesondere vorgesehen, die Produktstapel 8 relativ zur Schlauchfolie 16 korrekt zu positionieren, so dass die Lage der Produktstapel 8 relativ zu einer auf der Schlauchfolie 16 aufgebrachten Bedruckung und/oder relativ zu einer darin eingebrachten Perforation vorgegebene Randbedingungen erfüllt. Damit kann beispielsweise sichergestellt sein, dass eine durch eine Perforation in der Schlauchfolie 16 gebildete Zugriffsöffnung im Verpackungsbeutel auch geeignet relativ zum in den Verpackungsbeutel eingeschlagenen Produktstapel 8 positioniert ist.

Aus diesen und ggf. auch noch weiteren Gründen ergibt sich das Erfordernis einer register- oder taktgenauen Zuführung der Produktstapel 8 zur Verpackereinheit 2. Dies bedeutet, dass die Produktstapel 8 sowohl geeignet synchronisiert zum Anlagenprozess insgesamt als auch in korrektem (Register)Abstand zueinander der Verpackereinheit 2 zugeführt werden müssen. Das Fördersystem 10 ist gezielt dafür ausgelegt, diese Erfordernisse mit apparativ vergleichsweise gering gehaltenem Aufwand selbst für die mehrsträngige Zuführung der Produktstapel 8 aus der Mehrzahl der Staplereinheiten 6 zu erfüllen.

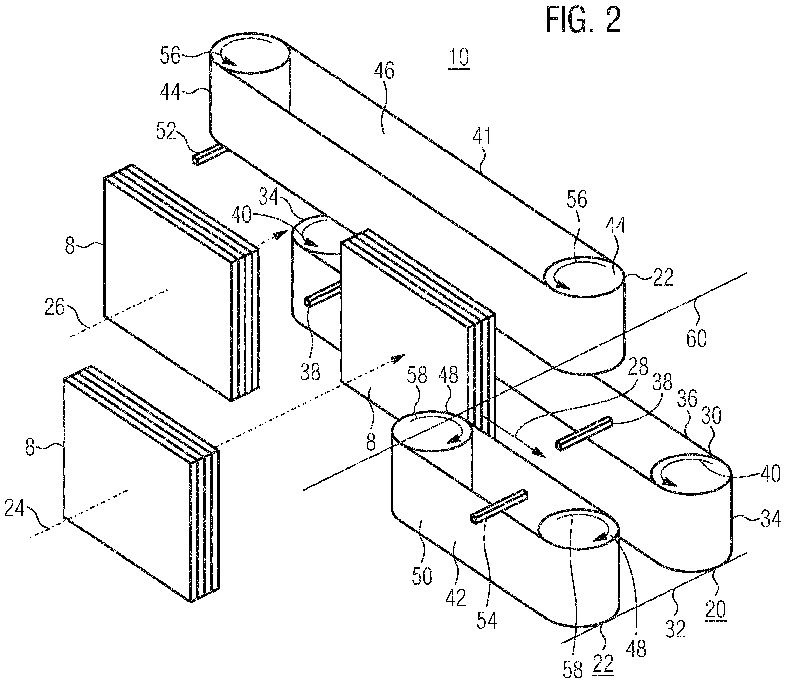

Dazu umfasst, wie dies der vergrößerten und grob vereinfachten schematischen Darstellung in FIG. 2 entnehmbar ist, das Fördersystem 10 für jede der Staplereinheiten 6 einen eigenen Förderer 20, 22, wobei der erste Förderer 20 der ersten Staplereinheit 6 und der zweite Förderer 22 der zweiten Staplereinheit 6 zugeordnet ist. Aus Gründen der Übersichtlichkeit sind dabei in FIG. 2 lediglich die für die Funktionsweise der vorliegenden Erfindung relevanten Komponenten schematisch dargestellt.

Dabei symbolisiert der Pfeil 24 den Produktstrom der von der ersten Staplereinheit 6 bereitgestellten Produktstapel 8, wobei der Produktstrom lediglich symbolisch für die angelieferte Sequenz der Produktstapel 8 zu verstehen ist; eine tatsächliche Anlieferung in linearer Richtung, wie durch den Pfeil 24 dargestellt, kann vorgesehen sein, dies ist aber nicht zwingend der Fall. Ebenso symbolisiert der Pfeil 26 den Produktstrom der von der zweiten Staplereinheit 6 bereitgestellten Produktstapel 8.

Die genannten Produktströme werden parallel nebeneinander dem Fördersystem 10 zugeführt. Dort wird die Förderrichtung der Produktstapel 8 um 90° gedreht, und die Produktstapel 8 werden in der durch den Pfeil 28 angedeuteten Transportrichtung aus dem Fördersystem 10 abgeführt. Im Fördersystem 10 werden die Produktströme dabei vereinigt, so dass beim Abtransport in der durch den Pfeil 28 angedeuteten Transportrichtung ein einheitlicher, vereinigter Produktstrom aus sämtlichen Produktstapeln 8 vorliegt. Der Ab- und Weitertransport der Produktstapel 8 aus dem Fördersystem 10 erfolgt im übrigen in einem geeignet gewählten und positionierten Kanalsystem, dessen Komponenten, Kanalwände und dergleichen in der FIG. aber aus Gründen der Übersichtlichkeit nicht dargestellt sind.

Um die genannten Erfordernisse hinsichtlich Registergenauigkeit und dergleichen zu erfüllen, ist der erste Förderer 20 einteilig ausgestaltet in dem Sinne, dass er in der durch den Pfeil 28 angedeuteten Transportrichtung des Produktstroms gesehen lediglich eine, im Ausführungsbeispiel als Riemenförderer ausgestaltete Fördereinheit 30 aufweist, die den vollständigen Transport der Produktstapel 8 von der Einbringstelle bis hin zur durch die Linie 32 angedeutete Übergabeposition an die nachfolgenden Komponenten bewerkstelligt. Die Fördereinheit 30 umfasst dabei einen über Walzen 34 geführten Förderriemen 36, an dem in geeignet gewähltem Abstand zueinander Mitnehmerstifte 38 angebracht sind. Jede der Walzen 34 ist jeweils mit einem nicht näher dargestellten ansteuerbaren oder programmierbaren Servoantrieb versehen, der sie in durch die Pfeile 40 symbolisierte Drehung versetzt. Hierdurch wird der Förderriemen 36 und mit diesem die Mitnehmerstifte 38 in Richtung der Transportrichtung bewegt. Durch den Antrieb über die genannten Servoantriebe kann die Transportgeschwindigkeit an die Erfordernisse des aktuellen Prozesszustandes angepasst werden.

Die Fördereinheit 30 ist dabei mit einem Bewegungsprofil ihrer Transportgeschwindigkeit beaufschlagt, das eine vergleichsweise schonende Behandlung der Produktstapel 8, insbesondere im Hinblick auf die mechanischen Belastungen, gewährleistet. Insbesondere ist dazu vorgesehen, dass die Fördereinheit 30 den jeweiligen Produktstapel 8 von der der Staplereinheit 6 nachfolgenden Ablegeposition mit einer zunächst vergleichsweise niedrigen Geschwindigkeit aufnimmt und ihn anschließend beschleunigt. Über eine entsprechende Einstellung der Transportgeschwindigkeit erfolgt sodann eine Anpassung an die relative Position im Produktionsablauf, um damit die erforderliche Registerhaltigkeit im Hinblick auf die Erfordernisse in der nachfolgenden Einschlagstation 14 zu gewährleisten. Anschließend wird der Produktstapel 8 mit einer an die Fördergeschwindigkeit der Verpackereinheit 2 angepassten Geschwindigkeit an diese abgegeben.

Dabei kann bedarfsweise auch der Einsatz zusätzlich vorgesehener, im Transportkanal für die Produktstapel 8 angeordneter Bremsmittel wie beispielweise Reibbremsen oder dergleichen vorgesehen sein, um auch die Geschwindigkeitsreduktion der Produktstapel 8 vor der Übergabe an nachfolgende Systeme mit niedrig gehaltener Belastung ausführen zu können. Der Einsatz zusätzlicher Bremssysteme ermöglicht darüber hinaus auch, den eigentlichen Transportkanal und seine Wandkomponenten für eine gering gehaltene Behinderung der Bewegung der Produktstapel 8 auszuführen, beispielsweise durch besonders glatt gehaltenen Kanalwände oder ausreichend breit bemessenen Kanalquerschnitt. Damit können die Produktstapel 8 weitegehend frei von seitlich angreifenden Bremskräften und dementsprechend belastungsfrei transportiert werden, insbesondere da ein eventuell erforderlicher Geschwindigkeitsabbau vor der Abgabe an die nachfolgenden Systeme zuverlässig über die Bremseinrichtung gewährleistet werden kann.

Der der weiteren Staplereinheit 6 zugeordnete zweite Förderer 22 ist hingegen mehrteilig, im Ausführungsbeispiel zweiteilig ausgeführt. Dazu umfasst der zweite Förderer 22 in Transportrichtung des Produktstroms gesehen eine Mehrzahl, im Ausführungsbeispiel zwei, hintereinander in der Art einer Reihenschaltung angeordnete Fördereinheiten 41, 42, auf die der vollständige Transport der Produktstapel 8 von der Abgabestelle der Stapeleinheit 6 bis hin zur durch die Linie 32 angedeutete Übergabeposition an die nachfolgenden Komponenten aufgeteilt ist. Auch die Fördereinheiten 41, 42 umfassen jeweils einen über Walzen 44 bzw. 48 geführten Förderriemen 46 bzw. 50, an dem in geeignet gewähltem Abstand zueinander Mitnehmerstifte 52 bzw. 54 angebracht sind. Jede der Walzen 44, 48 ist ebenfalls jeweils mit einem nicht näher dargestellten ansteuerbaren oder programmierbaren Servoantrieb versehen, der sie in durch die Pfeile 56, 58 symbolisierte Drehung versetzt. Hierdurch wird der jeweilige Förderriemen 46, 50 und mit diesem die Mitnehmerstifte 52, 54 in Richtung der Transportrichtung bewegt. Durch den Antrieb über die genannten Servoantriebe kann die Transportgeschwindigkeit an die Erfordernisse des aktuellen Prozesszustandes angepasst werden.

Die in Transportrichtung des Produktstroms gesehen erste dieser Fördereinheiten 41 ist räumlich gesehen oberhalb der Fördereinheit 30 und mit dieser teilweise überlappend angeordnet. Ihre Mitnehmerstifte sind dabei am unteren Rand des Förderriemens 46 angeordnet, wohingegen die Mitnehmerstifte 38 des Förderriemens 36 an dessen oberen Rand angeordnet sind. Dadurch ist erreicht, dass die Produktstapel 8 in jedem Fall in einer vergleichbaren Höhe zu Transportzwecken kontaktiert werden. Die Fördereinheit 41 ist hinsichtlich ihres Bewegungsprofils ähnlich wie die Fördereinheit 30 vornehmlich für eine vergleichsweise schonende Annahme und Beschleunigung des jeweiligen Produktstapels 8 bei der Übernahme von der Ablageposition der Staplereinheit 6 ausgelegt. Sie fördert den jeweiligen Produktstapel 8 bis zu einer durch die Linie 60 angedeuteten Übergabeposition, wo der Produktstapel 8 an die nachfolgende Fördereinheit 42 übergeben wird.

Die nachfolgende Fördereinheit 42, an die der jeweilige Produktstapel 8 von der vorangehenden Fördereinheit 40 übergeben wird, erfüllt prozesstechnisch die Funktion eines Puffer- und/oder Synchronisationselements, wobei die Fördergeschwindigkeit derart eingestellt wird, dass gerade auch in Abstimmung mit der Fördereinheit 30 der ersten Staplereinheit 6 eine register- und taktgenaue Vereinigung der Produktströme miteinander möglich ist.

### Bezugszeichenliste

- 1: Anlage
- 2: Verpackereinheit
- 4: Folienschlauch
- 6: Staplereinheit
- 8: Produktstapel
- 10: Fördersystem
- 12: Zuführschiene
- 14: Einschlagstation
- 16: Schlauchfolie
- 18: Formschuh
- 20: erster Förderer
- 22: zweiter Förderer
- 24, 26, 28: Pfeil
- 30: Fördereinheit
- 32: Linie
- 34: Walze
- 36: Förderriemen
- 38: Mitnehmerstifte
- 40: Pfeil
- 41, 42: Fördereinheit
- 44, 48: Walze
- 46, 50: Förderriemen
- 52, 54: Mitnehmerstifte
- 56, 58: Pfeil
- 60: Linie

## Patentansprüche

1. Anlage (1) zur Herstellung von verpackten Produktstapeln (8) von gefalzten oder ungefalzten Produktzuschnitten aus Papier, Zellstoff oder dergleichen, insbesondere von Hygieneprodukten, mit mindestens zwei produktstromseitig parallelgeschalteten Staplereinheiten (6) zum Bilden der Produktstapel (8) aus den Produktzuschnitten, denen produktstromseitig über ein Fördersystem (10) eine gemeinsame Verpackereinheit (2) nachgeschaltet ist, **dadurch gekennzeichnet, dass** das Fördersystem (10) einen ersten, einer der Staplereinheiten (6) zugeordneten mindestens einteiligen Förderer (20) und einen zweiten, einer zweiten Staplereinheit (6) zugeordneten mehrteiligen Förderer (22) umfasst.

2. Anlage (1) nach Anspruch 1, deren Fördersystem (10) als Bandfördersystem mit seitlich an den Produktstapeln (8) angreifenden Transportbändern ausgeführt ist.

3. Anlage (1) nach Anspruch 1 oder 2, deren zweiter Förderer (22) zweiteilig ausgeführt ist.

4. Anlage (1) nach einem der Ansprüche 1 bis 3, deren zweiter Förderer (22) mindestens zwei unabhängig voneinander ansteuerbare Fördereinheiten (41, 42) aufweist.

5. Anlage (1) nach einem der Ansprüche 1 bis 4, bei der der oder jeder Förderer (20, 22) antriebsseitig mit einem ansteuerbaren Servoantrieb versehen ist.

## Claims

1. Facility (1) for the production of packaged product stacks (8) of folded, or unfolded, product blanks made from paper, cellulose, or similar, in particular of hygiene products, with at least two stacking units (6), which are connected in parallel on the product flow side, for the formation of the product stacks (8) from the product blanks, downstream of which stacking units (6) a common packaging unit (2) is connected on the product flow side by way of a conveyor system (10), **characterised in that** the conveyor system (10) comprises a first conveyor (20), which is associated with one of the stacking units (6), and comprises at least one part, and a second conveyor (22), which is associated with a second stacking unit (6), and comprises a plurality of parts.

2. Facility (1) according to Claim 1, the conveyor system (10) of which is embodied as a belt conveyor system, with conveyor belts engaging laterally with the product stacks (8).

3. Facility (1) according to Claim 1 or 2, the second conveyor (22) of which is embodied in two parts.

4. Facility (1) according to one of the Claims 1 to 3, the second conveyor (22) of which has at least two conveyor units (41, 42) that can be controlled independently of one another.

5. Facility (1) according to one of the Claims 1 to 4, in which the, or each, conveyor (20, 22) is provided on the drive side with a servo drive that can be controlled.

## Revendications

1. Installation (1) pour la fabrication de piles de produits emballés (8) composées de découpe de produits pliées ou non pliées en papier, cellulose ou similaire, en particulier de produits hygiéniques, comportant au moins deux unités d'empilement positionnées en parallèle au niveau du flux de produits (6) pour constituer les piles de produits (8) à partir des découpes de produits et en aval desquels, au niveau du flux de produits, grâce à un système de transport (10), une unité d'emballage commune (2) est installée, **caractérisée en ce que**
le système de transport (10) comprend un premier convoyeur (20) formé d'au moins une pièce et associé à une des unités d'empilement (6) et un second convoyeur (22) composé de plusieurs pièces et associé à une seconde unité d'empilement (6).

2. Installation (1) selon la revendication 1, dont le système de transport (10) est réalisé sous forme d'un système de convoyage à bande doté de bandes transporteuses empiétant latéralement sur les piles de produits (8).

3. Installation (1) selon la revendication 1 ou 2, dont le second convoyeur (22) est réalisé en deux pièces.

4. Installation (1) selon une des revendications 1 à 3, dont le second convoyeur (22) présente au moins deux unités de convoyage contrôlables indépendamment l'une de l'autre (41, 42).

5. Installation (1) selon une des revendications 1 à 4, dans lequel le ou chaque convoyeur (20, 22) est pourvu au niveau propulsion d'une servocommande contrôlable.
